# EUROPÄISCHE PATENTSCHRIFT

(11) **EP 0 918 765 B1**
(45) Veröffentlichungstag und Bekanntmachung des Hinweises auf die Patenterteilung: **15.01.2003**
(21) Anmeldenummer: 97929276.0
(22) Anmeldetag: 24.06.1997
(51) Int. Cl.: C07D 307/88

(54) **VERFAHREN ZUR HERSTELLUNG VON PHTHALIDEN**
PROCESS FOR PREPARING PHTHALIDES
PROCEDE DE PREPARATION DE PHTHALIDES

(30) Priorität: 10.07.1996 DE 19627697
(43) Veröffentlichungstag der Anmeldung: 02.06.1999
(73) Patentinhaber: BASF AKTIENGESELLSCHAFT, 67056 Ludwigshafen (DE)
(72) Erfinder: MASSONNE, Klemens, D-67368 Westheim (DE); BECKER, Rainer, D-67098 Bad Dürkheim (DE); REIF, Wolfgang, D-67227 Frankenthal (DE); WULFF-DÖRING, Joachim, D-67227 Frankenthal (DE)
(74) Vertreter: Fitzner, Uwe, Dr.
(86) Internationale Anmeldenummer: EP9703303
(87) Internationale Veröffentlichungsnummer: WO98001438

(56) Entgegenhaltungen:
- EP-A- 0 542 037
- DE-A- 2 544 761
- DE-A- 3 201 300

## Beschreibung

Die vorliegende Erfindung betrifft ein Verfahren zur Herstellung von Phthaliden durch katalytische Hydrierung von Phthalsäureanhydriden mit Hilfe von mit Metallen der 1. Nebengruppe, 6. Nebengruppe und/oder 7. Nebengruppe des Periodensystems dotierten Raney-Nickelkatalysatoren.

Aus DE-C-28 03 319 ist es bekannt, Phthalid durch katalytische Hydrierung von Phthalsäureanhydrid in der Gasphase mit Kupfer und Aluminium enthaltenden Katalysatoren durchzuführen. Dieses Verfahren erfordert jedoch einen erhöhten Aufwand bei der Isolierung des Reaktionsproduktes durch mehrere Kondensationsstufen und einer nachgeschalteten Abgaswäsche. In EP-A-542 037 wird weiter ein Verfahren zur Herstellung von Phthaliden durch katalytische Hydrierung von Phthalsäureanhydrid an Festbettkatalysatoren beschrieben, das bei diskontinuierlicher Arbeitsweise hohe Aufwandmengen an Katalysator und lange Hydrierzeiten erfordert, während bei kontinuierlicher Arbeitsweise hohe Drücke erforderlich sind, um einen vollständigen Umsatz zu erreichen.

Aus US-A-4,485,246 und US-A-3,957,825 ist es außerdem bekannt, daß man Phthalid aus Phthalsäureanhydrid durch Hydrierung mit homogenen Rutheniumkatalysatoren herstellen kann. Bei diesen Verfahren ist die Katalysatorrückgewinnung schwierig.

In EP-B-89 417 ist weiter die katalytische Hydrierung von Phthalsäureanhydrid zu Phthalid mit Hilfe eines auf einem Trägermaterial fixierten Nickelkatalysators beschrieben, wobei die Verwendung von Benzoesäuremethylester als Lösungsmittel zwingend vorgeschrieben ist. Das Verfahren hat den Nachteil hoher Aufwandmengen an Katalysator und langer Reaktionszeiten.

Aus Houben/Weyl, "Methoden der organischen Chemie", 6/2 (1963), Seiten 732 bis 733 ist weiter die Hydrierung von Phthalsäureanhydrid zu Phthalid mit Hilfe von Raney-Nickel als Katalysator bekannt, wobei bei einem Wasserstoffdruck von 165 bar in Ethanol als Lösungsmittel nur eine Ausbeute an Phthalid von 73 % erhalten wird.

Der vorliegenden Erfindung lag daher die Aufgabe zugrunde, den zuvor genannten Nachteilen bei der Verwendung der bekannten Katalysatoren bei der katalytischen Hydrierung von Phthalsäureanhydriden abzuhelfen. Demgemäß wurde ein neues und verbessertes Verfahren zur Herstellung von Phthaliden der allgemeinen Formel I, 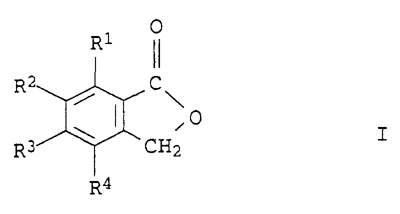 in der R¹, R², R³ und R⁴ unabhängig voneinander Wasserstoff, C₁-C₄-Alkyl oder C₁-C₄-Alkoxy bedeuten, durch Hydrierung von Phthalsäureanhydriden der allgemeinen Formel II, 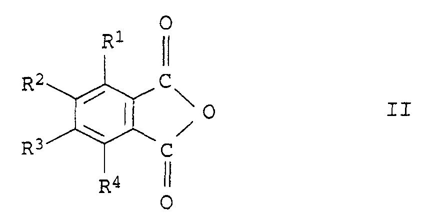 in der die Reste R¹, R², R³ und R⁴ die oben genannten Bedeutungen haben, mit Hilfe von Hydrierkatalysatoren gefunden, welches dadurch gekennzeichnet ist, daß man die Hydrierung mit Hilfe von mit Metallen der 1. Nebengruppe, der 6. Nebengruppe und/oder 7. Nebengruppe des Periodensystems dotierten Raney-Nickelkatalysatoren durchführt.

Nach dem erfindungsgemäßen Verfahren läßt sich die Hydrierung von den Phthalsäureanhydriden zu den Phthaliden bei geringen Aufwandmengen der Katalysatoren mit guter Raum-Zeit-Ausbeute und hoher Selektivität durchführen. Die erfindungsgemäß zu verwendenden Katalysatoren weisen gegenüber herkömmlichem Raney-Nickel eine deutlich erhöhte Aktivität auf. Dies führt zu milderen Reaktionsbedingungen bzw. bei Anwendung der bei der Verwendung von nicht dotiertem Raney-Nickel üblichen Reaktionsbedingungen zu schnellerer Hydrierung und damit zu höheren Raum-Zeit-Ausbeuten.

Gegenüber den gemäß dem in EP-A-542 037 beschriebenen Verfahren zu verwendenden Katalysatoren weisen die erfindungsgemäß zu verwendenden Katalysatoren ebenfalls eine deutlich erhöhte Aktivität auf. Während gemäß dem bekannten Verfahren bei 12 Stunden Hydrierzeit und 260 bar Druck mit Mengen von 10 % Katalysator, bezogen auf das eingesetzte Phthalsäureanhydrid, in der Regel trotz Anwendung hoher Reaktionstemperaturen von 150 bis 250°C nur unvollständige Umsätze erreicht werden, wird nach dem erfindungsgemäßen Verfahren unter milderen Reaktionsbedingungen in kürzerer Zeit ein praktisch vollständiger Umsatz erzielt.

Die katalytische Hydrierung wird im allgemeinen bei Temperaturen von 50 bis 400°C, vorzugsweise 100 bis 250°C, insbesondere 140 bis 220°C und Drücken von 1 bis 400 bar, vorzugsweise 5 bis 300 bar, insbesondere 5 bis 200 bar, besonders vorteilhaft 30 bis 120 bar durchgeführt.

Als Reaktoren können übliche Hydrierreaktoren, z.B. Autoklaven oder Rohrreaktoren, verwendet werden.

Als Hydrierkatalysatoren werden für das erfindungsgemäße Verfahren Raney-Nickelkatalysatoren, die mit Metallen der 1. Nebengruppe des Periodensystems, im allgemeinen Silber, Kupfer, vorzugsweise Kupfer, mit Metallen der 6. Nebengruppe des Periodensystems, im allgemeinen Chrom, Molybdän oder Wolfram, vorzugsweise Molybdän und/oder mit Metallen der 7. Gruppe des Periodensystems, im allgemeinen Mangan, Rhenium, vorzugsweise Rhenium dotiert sind. Im allgemeinen beträgt der Gehalt der erfindungsgemäß zu verwendenden Katalysatoren an den zur Dotierung zugesetzten Metallen 0,01 bis 10 Gew.-%, vorzugsweise 0,1 bis 5 Gew.-%, bezogen auf das in den Katalysatoren enthaltene Nickel.

Die erfindungsgemäß zu verwendenden Katalysatoren werden in üblicher Weise (DE-A-25 44 761) hergestellt, beispielsweise indem man Nickel, die Dotierungsmetalle und mit Lauge herauslösbare Stoffe, vorzugsweise Aluminium und/oder Silizium, legiert und die laugelöslichen Stoffe anschließend mit Lauge, wie Natronlauge herausgelöst.

Die Hydrierkatalysatoren können z.B. als Trägerkatalysatoren oder suspendierte Katalysatoren eingesetzt werden.

Die Hydrierung kann ohne Lösungsmittel durchgeführt werden, wobei das Phthalsäureanhydrid in geschmolzener Form eingesetzt wird.

In der Regel wird die Hydrierung unter Verwendung eines Lösungsmittels durchgeführt. Geeignete Lösungsmittel sind beispielsweise Ether wie Tetrahydrofuran, Dioxan, Glykolether, Ester wie Essigsäuremethylester und Methylbenzoat, Lactone wie Butyrolacton oder Phthalid, vorzugsweise das bei der Hydrierung gebildete Phthalid, Alkohole wie Methanol, Ethanol, Propanol, Butanol, Kohlenwasserstoffe oder Gemische dieser Lösungsmittel.

Das Gewichtsverhältnis von dem zu hydrierenden Phthalsäureanhydrid zu dem verwendeten Lösungsmittel beträgt im allgemeinen 1000:1 bis 1:1000, vorzugsweise 500:1 bis 1:500, insbesondere 200:1 bis 1:200.

Zweckmäßig wird man die erfindungsgemäße Umsetzung abbrechen, sobald alles eingesetzte Phthalsäureanhydrid hydriert ist. Der Zeitpunkt für den Abbruch wird z.B. durch Bestimmung des noch vorhandenen Phthalsäureanhydrids, beispielsweise durch GasChromatographie oder aus dem zeitlichen Verlauf der Wasserstoffaufnahme ermittelt.

Die Aufarbeitung des Reaktionsproduktes erfolgt nach üblichen Methoden, vorzugsweise durch Destillation.

In den Verbindungen I und II bedeuten R¹, R², R³ und R⁴ unabhängig voneinander Wasserstoff; C₁-C₄-Alkyl wie Methyl, Ethyl, n-Propyl, iso-Propyl, n-Butyl, iso-Butyl, sec.-Butyl, tert.-Butyl, bevorzugt Methyl und Ethyl, insbesondere Methyl; C₁-C₄-Alkoxy wie Methoxy, Ethoxy, n-Propoxy, iso-Propoxy, n-Butoxy, iso-Butoxy, sec.-Butoxy, tert.-Butoxy, bevorzugt Methoxy und Ethoxy, insbesondere Methoxy. Besonders bevorzugt bedeuten alle Substituenten R¹, R², R³ und R⁴ Wasserstoff.

Die als Ausgangsstoffe verwendeten Phthalsäureanhydride II sind allgemein bekannt, wie das Phthalsäureanhydrid, oder können nach bekannten Verfahren erhalten werden (J. of Org. Chemistry 51, 3439-3446 (1986); Synthesis 223-224 (1985)).

Die Phthalide I eignen sich z.B. als Ausgangsstoff für die Synthese von Pflanzenschutzmitteln.

Die Erfindung wird durch folgende Beispiele verdeutlicht.

### Beispiel 1

In einem 0,5 1-Rührautoklav mit Begasungsrührer werden 88,8 g Phthalsäureanhydrid, 207,2 g Phthalid als Lösungsmittel und 0,9 g Katalysator unter Stickstoff vorgelegt und die Mischung Phthalsäureanhydrid/Phthalid bei 120°C geschmolzen. Der Rührer wird mit einer Drehzahl von 500 Upm eingeschaltet, der Stickstoff durch mehrmaliges Aufdrücken von Wasserstoff auf einen Druck von 5 bar und Entspannen gegen Wasserstoff ausgetauscht. Der Reaktorinhalt wird auf 180°C erwärmt. Es wird Wasserstoff auf einen Druck von 40 bar aufgepreßt und bis zur Druckkonstanz hydriert. Der Umsatz an Phthalsäureanhydrid ist dann nahezu quantitativ.

Die Versuchsbedingungen und Ergebnisse sind in der folgenden Tabelle zusammengefaßt:

| Versuchs-Nr. | Katalysator | Dotierung in Gew.-% bezogen auf Nickelgehalt des Katalysators | Hydrierzeit min | Selektivität % |
|---|---|---|---|---|
| 1a | Raney-Nickel (Katalysator BK 113 W; Degussa) | 1 % Molybdän | 115 | 87 |
| 1b | Raney-Nickel (Katalysator BK 111 W; Degussa) | 1 % Molybdän | 79 | 89 |
| 1c | Raney-Nickel (Katalysator Actimet C; Doduco) | 1 % Molybdän | 103 | 89 |
| 1d | Raney-Nickel | 1 % Kupfer | 70 | 86 |
| 1e | Raney-Nickel | 1 % Rhenium | 30 | 88 |

### Vergleichsbeispiele (nicht erfindungsgemäß)

Die Versuche werden wie in Beispiel 1 beschrieben durchgeführt, wobei jedoch nicht dotierte Raney-Nickel-Katalysatoren bzw. nicht erfindungsgemäß dotierte Raney-Nickel-Katalysatoren verwendet werden.

Die Versuchsbedingungen und Ergebnisse sind in der folgenden Tabelle zusammengefaßt:

| Versuchs-Nr. | Katalysator | Dotierung | Hydrierzeit min | Selektivität % |
|---|---|---|---|---|
| 2a | Raney-Nickel (B 113 CW; Degussa) | keine | 208 | 85 |
| 2b | Raney-Nickel B 113 Z; Degussa) | keine | 203 | 84 |
| 2c | Raney-Nickel (Actimet S; Doduco) | keine | 177 | 80 |
| 2d | Raney-Nickel (Actimet MA; Doduco) | keine | 145 | 86 |
| 2e | Raney-Nickel BM 113 Z; Degussa) | 1 % Eisen | 338 | 87 |

Bei der Verwendung der nicht erfindungsgemäßen Raney-Nickel-Katalysatoren sind deutlich verlängerte Hydrierzeiten erforderlich, um einen vergleichbaren Umsatz zu erzielen, wobei in der Regel auch noch die Selektivität geringer ist.

## Patentansprüche

1. Verfahren zur Herstellung von Phthaliden der allgemeinen Formel I, 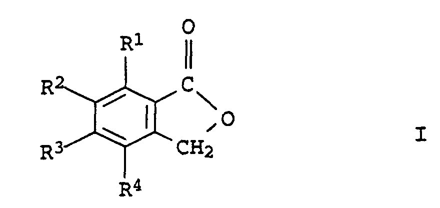 in der R¹, R², R³ und R⁴ unabhängig voneinander Wasserstoff, C₁-C₄-Alkyl oder C₁-C₄-Alkoxy bedeuten, durch Hydrierung von Phthalsäureanhydriden der allgemeinen Formel II, 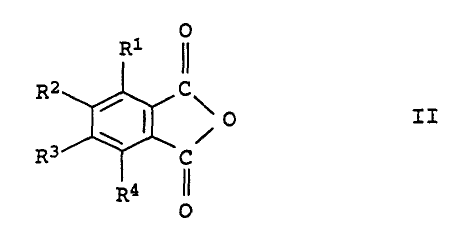 in der die Reste R¹, R², R³ und R⁴ die oben genannten Bedeutungen haben, mit Hilfe von Hydrierkatalysatoren, **dadurch gekennzeichnet, daß** man die Hydrierung mit Hilfe von mit Metallen der 1. Nebengruppe, der 6. Nebengruppe und/oder 7. Nebengruppe des Periodensystems dotierten Raney-Nickelkatalysatoren durchführt.

## Claims

1. A process for preparing a phthalide of the general formula I 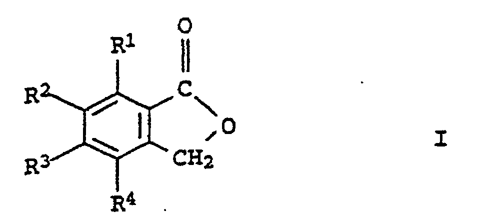 where R¹, R², R³ and R⁴ are each independently of the others hydrogen, C₁-C₄-alkyl or C₁-C₄-alkoxy, by hydrogenation of a phthalic anhydride of the general formula II 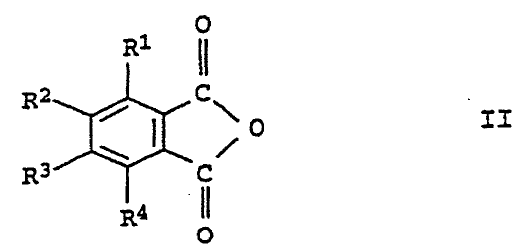 where R¹, R², R³ and R⁴ are each as defined above, with the aid of a hydrogenation catalyst, which comprises performing the hydrogenation with the aid of a Raney nickel catalyst doped with metals of subgroup 1, subgroup 6 and/or subgroup 7 of the Periodic Table.

## Revendications

1. Procédé pour la préparation de phtalides de formule générale I 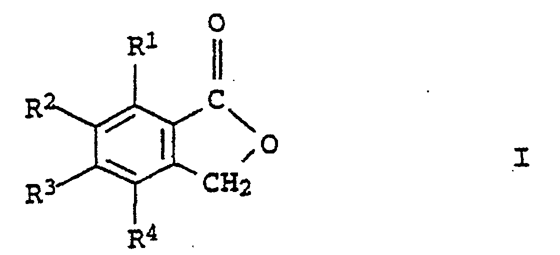 dans laquelle R¹, R², R³ et R⁴ représentent, indépendamment les uns des autres, un atome d'hydrogène, un groupe alkyle en C₁-C₄ ou alcoxy en C₁-C₄, par hydrogénation d'anhydrides phtaliques de formule générale II 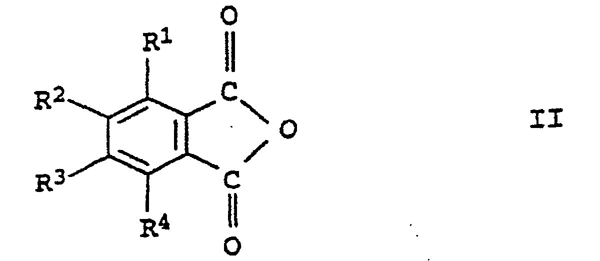 dans laquelle les radicaux R¹, R², R³ et R⁴ ont les significations indiquées, à l'aide de catalyseurs d'hydrogénation, **caractérisé en ce qu'**on effectue l'hydrogénation à l'aide de catalyseurs à base de nickel de Raney, dopé avec des métaux du groupe IB, du groupe VIB et/ou du groupe VIIB de la classification périodique.
